# EUROPEAN PATENT SPECIFICATION

(11) **EP 3 245 940 B1**
(45) Date of publication and mention of the grant of the patent: **03.04.2019**
(21) Application number: 16197663.4
(22) Date of filing: 08.11.2016
(51) Int. Cl.: A61B 1/313, A61B 1/015, A61B 1/018, A61B 1/00

(54) **SPINAL ENDOSCOPE WITH NOVEL ELONGATE RIGID TUBE**
SPINALENDOSKOP MIT NEUARTIGEM LÄNGLICHEM STARREN ROHR
ENDOSCOPE VERTÉBRAL À NOUVEAU TUBE RIGIDE ALLONGÉ

(30) Priority: 18.05.2016 KR 20160060686
(43) Date of publication of application: 22.11.2017
(73) Proprietor: Techcord Co., Ltd, Daejeon 34620 (KR)
(72) Inventor: CHOI, Young Kyu, 34964 Daejeon (KR)
(74) Representative: Zardi, Marco

(56) References cited:
- WO-A1-93/20742
- WO-A1-2010/048208
- CN-A- 104 887 295
- US-A1- 2013 131 445

## Description

### BACKGROUND

### Field of the Present Disclosure

The present disclosure relates to a spinal endoscope, and, more particularly, to a spinal endoscope with an elongate rigid tube having a novel working channel.

### Discussion of Related Art

Spinal endoscopy is a procedure in which a small endoscope is passed up through the tailbone into the epidural space. This allows for direct video imaging of the inside of the spinal canal. Spinal endoscopy is also known as epiduroscopy because the endoscope is looking into the epidural space. During a spinal endoscopy, an attempt to remove some of the scar tissue or adhesions from around trapped nerves. The spinal endoscopy may allow medications to better reach the affected areas, especially the spinal nerve roots.

Endoscopic spine surgery is evolving rapidly due to improvements in surgical technique, endoscope design, and instrumentation. In an experienced surgeon's hands, the endoscopic foraminal approach can be utilized for most lumbar disc herniations and for the diagnosis and treatment of degenerative conditions of the lumbar spine. The advantage of the foraminal endoscopic technique is the ability to reach, visualize, and treat intradiscal and foraminal pathologic lesions without destabilizing the posterior muscle column and facets. The learning curve is steep, but once mastered, the surgeon is able to reach any pathologic lesion in the foramen, including noncontained disc herniations, foraminal stenosis, foraminal osteophytosis, facet cysts, and annular tears.

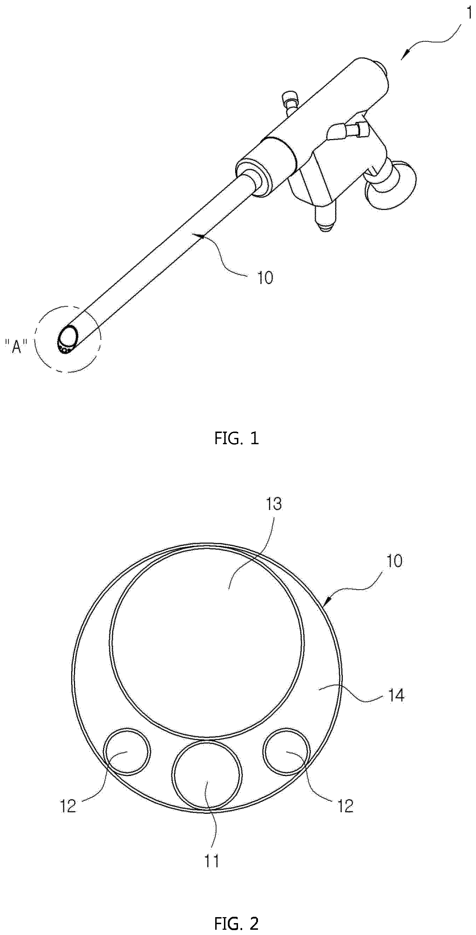

FIG. 1 is a perspective of a conventional spinal endoscope 1. The spinal endoscope may have an elongate rigid tube 10. FIG. 2 is an enlarged cross-sectional view of the elongate rigid tube 10. As shown in FIG. 2, the elongate rigid tube 10 may receive therein a lens 11 to allow observation of a tissue, an irrigation channel 12 to allow injection of a cleaning liquid such as saline solution to remove bloods to secure a view for surgery, and a working channel 13 to allow a surgery-tool such as a forcep or punch to be guided to a target location. Further, besides the lens 11 or channels 12, 13, an optical fiber 14 is disposed to allow irradiation of light beams.

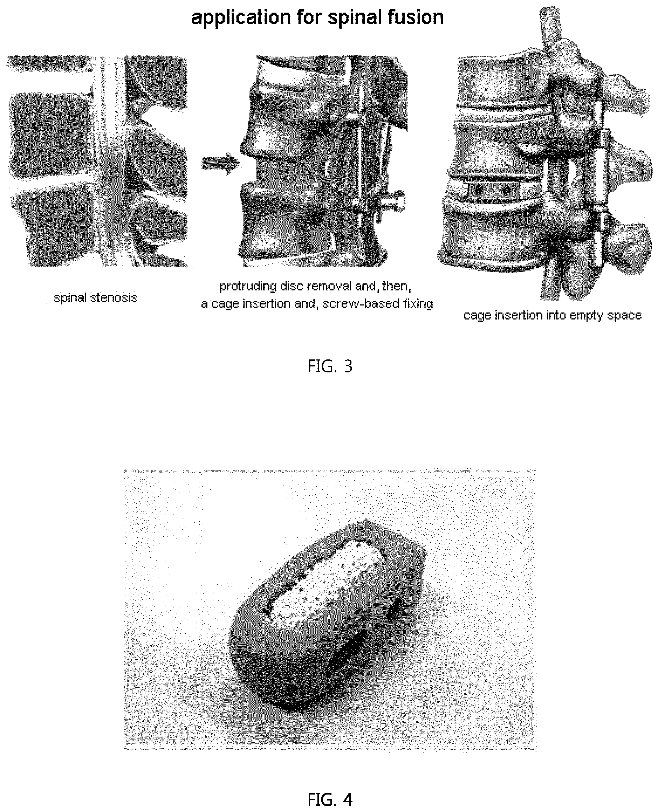

Recently, for a minimal invasion, the elongate rigid tube 10 of the conventional spinal endoscope may have a smaller size. Thus, the diameter of the cross-section of the working channel may be smaller. Thus, it may be difficult to use the surgery-tool such as a forcep or punch. In particular, when performing a surgical treatment for a spinal stenosis, as shown in FIG. 3, the degenerative changed bone, ligament tissue, protruding disc are removed and, then, a cage as shown in FIG. 4 as a disc replacement is inserted into the disc removal space. Then, in order to firmly secure the spine, a spinal fusion is carried out wherein a screw is fixed to the spinal bone. However, since the elongate rigid tube 10 of the conventional spinal endoscope has a smaller size, it may be difficult to carry out the spinal fusion. Thus, to carry out the spinal fusion, a larger target area should be subjected to incision. This may not achieve the minimal invasion which is intended initially.

A prior art document may be as follows: Patent document 1 Japanese utility model registration NO. 3176713; and Korean patent NO. 10-0900991. CN 104887295 A, WO 1993/020742 A1, WO 2010/048208 A1, and US 2013/0131445 A1 disclose endoscopes according to state of the art.

### SUMMARY

The invention is defined in independent claim 1 and the dependent claims 2 - 7.

The present disclosure is to provide a spinal endoscope having a novel rigid tube structure, wherein although, for a minimal invasion, an elongate rigid tube of a spinal endoscope has a small cross-section, an endoscopic spinal surgery may suffice to complete a surgery for a target spinal portion.

Further, the present disclosure is to provide a spinal endoscope having a novel rigid tube structure, wherein an endoscopic spinal surgery may suffice to complete an artificial disk replacement or spinal fusion.

The purpose of the present disclosure may be not limited to the above purpose. Other purposes thereof may be apparent from the following detailed description.

In one aspect of the present disclosure, there is provided a spinal endoscope comprising an imaging device and an elongate rigid tube coupled to the imaging device, wherein the tube receives therein: a lens coupled to the imaging device to allow observation of a tissue; at least one irrigation channel to allow injection of a cleaning liquid to secure a view for surgery; a working channel to allow passage of a surgery-tool therethrough to a target location, and an optical fiber to allow irradiation of light beams, the spinal endoscope being characterized in that the working channel includes a first portion with a circular arc cross-sectional shape face-contacting and partially corresponding to an inner circumference of the elongate rigid tube, and a second portion with a linear cross-sectional shape, wherein the second portion connects both ends of the first portion.

In one implementation of the spinal endoscope, a length of the first portion is at least about 70% of a total length of the inner circumference of the elongate rigid tube.

In one implementation of the spinal endoscope, a single lens is sandwiched between two irrigation channels.

In one implementation of the spinal endoscope, the cross-sectional area of the working channel is larger than a sum of the cross-sectional areas of the lens, the irrigation channel, and the optical fiber.

In one implementation of the spinal endoscope, the lens contacts both irrigation channels with the lens being sandwiched therebetween.

In one implementation of the spinal endoscope, the lens has a circular cross-sectional shape.

In one implementation of the spinal endoscope, the irrigation channel has a circular cross-sectional shape.

In one implementation of the spinal endoscope, the lens has a tangential contact with an inner circumference of the elongate rigid tube.

In one implementation of the spinal endoscope, the irrigation channel has a tangential contact with an inner circumference of the elongate rigid tube.

In one implementation of the spinal endoscope, the lens and irrigation channels all have the tangential contact with the inner circumference of the elongate rigid tube.

In one implementation of the spinal endoscope, the cross-sectional area of the working channel is at least about 80 % of the cross-sectional area of the elongate rigid tube.

In one implementation of the spinal endoscope, a circular structure with the largest diameter among the circular structures such as the lens and irrigation channels has a tangential contact with the linear second portion of the working channel.

In one implementation of the spinal endoscope, lens has the tangential contact with the linear second portion of the working channel.

In one implementation of the spinal endoscope, he working channel allows passage of a cage therethrough.

The novel working channel may allow faster and easier passage of a surgery-tool such as a forcep or punch, etc. therethrough to a target location when performing an endoscopic spinal surgery using the spinal endoscope. This may lead to improved success rate and/or shortened completion time of endoscopic spinal surgery.

The working channel may have a non-circular cross-sectional shape. By comparison, the cross-sectional area of the working channel may be at least about 200 % of the cross-sectional area of the working channel in the conventional approach as shown in FIG. 2 when the elongate rigid tubes in the present and conventional approaches have the same cross-sectional area.

Further, the lens and irrigation channels all have the tangential contact with the inner circumference of the elongate rigid tube. Thus, the space for the working channel may be sufficient. In this way, the cross-sectional area of the working channel may be larger.

### BRIEF DESCRIPTION OF THE DRAWINGS

The accompanying drawings, which are incorporated in and form a part of this specification and in which like numerals depict like elements, illustrate embodiments of the present disclosure and, together with the description, serve to explain the principles of the disclosure.
FIG. 1 is a perspective of a conventional spinal endoscope having an elongate rigid tube.
FIG. 2 is an enlarged cross-sectional view of a portion "A" in the elongate rigid tube in FIG. 1.
FIG. 3 illustrates a process of a spinal fusion.
FIG. 4 is a perspective of one example of a cage as an artificial disc replacement.
FIG. 5 is an enlarged cross-sectional view of a novel elongate rigid tube of a spinal endoscope in accordance with the present disclosure.

For simplicity and clarity of illustration, elements in the figures are not necessarily drawn to scale. The same reference numbers in different figures denote the same or similar elements, and as such perform similar functionality. Also, descriptions and details of well-known steps and elements are omitted for simplicity of the description. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

### DETAILED DESCRIPTIONS

Examples of various embodiments are illustrated and described further below. It will be understood that the description herein is not intended to limit the claims to the specific embodiments described. On the contrary, it is intended to cover alternatives, modifications, and equivalents as may be included within the spirit and scope of the present disclosure as defined by the appended claims.

Example embodiments will be described in more detail with reference to the accompanying drawings. The present disclosure, however, may be embodied in various different forms, and should not be construed as being limited to only the illustrated embodiments herein. Rather, these embodiments are provided as examples so that this disclosure will be thorough and complete, and will fully convey the aspects and features of the present disclosure to those skilled in the art.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section, without departing from the spirit and scope of the present disclosure.

It will be understood that when an element or layer is referred to as being "connected to", or "coupled to" another element or layer, it can be directly on, connected to, or coupled to the other element or layer, or one or more intervening elements or layers may be present. In addition, it will also be understood that when an element or layer is referred to as being "between" two elements or layers, it can be the only element or layer between the two elements or layers, or one or more intervening elements or layers may also be present.

Spatially relative terms, such as "beneath," "below," "lower," "under," "above," "upper," and the like, may be used herein for ease of explanation to describe one element or feature's relationship to another element s or feature s as illustrated in the figures. It will be understood that the spatially relative terms are intended to encompass different orientations of the device in use or in operation, in addition to the orientation depicted in the figures. For example, if the device in the figures is turned over, elements described as "below" or "beneath" or "under" other elements or features would then be oriented "above" the other elements or features. Thus, the example terms "below" and "under" can encompass both an orientation of above and below. The device may be otherwise oriented for example, rotated 90 degrees or at other orientations, and the spatially relative descriptors used herein should be interpreted accordingly.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, s, operations, elements, and/or components, but do not preclude the presence or addition of one or more other features, integers, s, operations, elements, components, and/or portions thereof. As used herein, the term "and/or" includes any and all combinations of one or more of the associated listed items. Expression such as "at least one of" when preceding a list of elements may modify the entire list of elements and may not modify the individual elements of the list.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

In the following description, numerous specific details are set forth in order to provide a thorough understanding of the present disclosure. The present disclosure may be practiced without some or all of these specific details. In other instances, well-known process structures and/or processes have not been described in detail in order not to unnecessarily obscure the present disclosure.

As used herein, the term "substantially," "about," and similar terms are used as terms of approximation and not as terms of degree, and are intended to account for the inherent deviations in measured or calculated values that would be recognized by those of ordinary skill in the art. Further, the use of "may" when describing embodiments of the present disclosure refers to "one or more embodiments of the present disclosure."

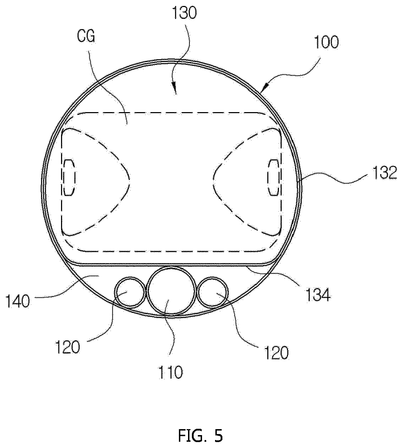

FIG. 5 is an enlarged cross-sectional view of a novel elongate rigid tube of a spinal endoscope in accordance with the present disclosure. The spinal endoscope in accordance with the present disclosure may be the same as the spinal endoscope 1 as shown in FIG. 1 except for a novel configuration of the elongate rigid tube 100. Thus, the present elongate rigid tube 100 receives therein a lens 110, an irrigation channel 120, a working channel 130 and an optical fiber 140. The lens 110 may allow observation of a tissue. The irrigation channel 120 may allow injection of a cleaning liquid such as saline solution to remove bloods to secure a view for surgery. The working channel 130 may allow a surgery-tool such as a forcep or punch to be guided to a target location. Further, besides the lens 110 or channels 120, 130, the optical fiber 140 is disposed to allow irradiation of light beams.

In accordance with the present disclosure, the single lens 110 is sandwiched between two irrigation channels 120 to clean around the lens 110.

The elongate rigid tube 100 may have the working channel 130 to allow a surgery-tool such as a forcep or punch to be guided to a target location and/or to allow passage of a cage as an artificial disc replacement for the spinal fusion. In accordance with the present disclosure, the cross-sectional area of the working channel 130 is larger than a sum of the cross-sectional areas of the lens 110, irrigation channel 120, and optical fiber 140. A remaining space excluding the spaces of the lens 110, irrigation channel 120, and working channel 130 may be filled with the optical fiber 140 to irradiate the target portion of the spine.

In accordance with the present disclosure, the lens 110 contacts both irrigation channels 120 with the lens 110 being sandwiched therebetween. The lens 110 may have a circular cross-sectional shape. Each of the irrigation channels 120 may have a circular cross-sectional shape. The lens 110 has a tangential contact with an inner circumference of the elongate rigid tube 100. Each of the irrigation channels 120 has a tangential contact with an inner circumference of the elongate rigid tube 100.

In accordance with the present disclosure, the working channel 130 has a first portion 132 with a circular arc cross-sectional shape face-contacting and partially corresponding to an inner circumference of the elongate rigid tube 100, and a second portion 134 with a linear cross-sectional shape, wherein the second portion 134 connects both ends of the first portion 132.

In the conventional approach as shown in FIG. 2, the lens and irrigation channels are spaced away from each other. However, In accordance with the present disclosure, the lens 110 and irrigation channels 120 contact each other. Further, the lens 110 and irrigation channels 120 all have the tangential contact with the inner circumference of the elongate rigid tube 100. Thus, the space for the working channel 130 may be sufficient. In this way, the cross-sectional area of the working channel 130 may be larger.

In one embodiment, the working channel 130 has a first portion 132 with a circular arc cross-sectional shape face-contacting and partially corresponding to an inner circumference of the elongate rigid tube 100, and a second portion 134 with a linear cross-sectional shape, wherein the second portion 134 connects both ends of the first portion 132. In this connection, the length of the first portion 132 with a circular arc cross-sectional shape may be at least about 70% of the total length of the inner circumference of the elongate rigid tube 100. In this way, the cross-sectional area of the working channel 130 may be at least about 80% of the cross-sectional area of the elongate rigid tube 100.

Further, in one embodiment, the circular structure with the largest diameter among the circular structure such as the lens 110 and irrigation channels 120 may have the tangential contact with the linear second portion 134 of the working channel 130. In one example, as shown in FIG. 5, the circular structure with the largest diameter is the lens 110. Thus, the lens 110 has the tangential contact with the linear second portion 134 of the working channel 130. In this way, the cross-sectional area of the working channel 130 may be maximized.

The working channel 130 has a non-circular cross-sectional shape. By comparison, the cross-sectional area of the working channel 130 may be at least about 200 % of the cross-sectional area of the working channel 30 in the conventional approach as shown in FIG. 2 when the elongate rigid tubes 10 and 100 have the same cross-sectional area.

This novel working channel 130 may allow the passage of a surgery-tool such as a forcep or punch, etc. therethrough to a target location when performing an endoscopic spinal surgery using the spinal endoscope 1. This may further give high freedom of selection of the surgery-tool. Further, the novel working channel 130 may allow faster and easier passage of a surgery-tool such as a forcep or punch, etc. therethrough to a target location when performing an endoscopic spinal surgery using the spinal endoscope 1. This may lead to improved success rate and/or shortened completion time of endoscopic spinal surgery.

In particular, when performing an artificial disk replacement or spinal fusion using the spinal endoscope 1, the novel working channel 130 may allow passage of a larger cage (CG) as shown by a dashed line in FIG. 5 therethrough without interference with the muscle or nerve tissue. In this way, without requiring of larger incision of the to-be-treated portion, the success of the endoscopic spinal surgery using the spinal endoscope 1 may be guaranteed. That is, the very reason for the endoscopic spinal surgery, the minimal invasion may be achieved.

The above description is not to be taken in a limiting sense, but is made merely for the purpose of describing the general principles of exemplary embodiments, and many additional embodiments of this disclosure are possible. It is understood that no limitation of the scope of the disclosure is thereby intended. The scope of the disclosure should be determined with reference to the claims. Reference throughout this specification to "one embodiment," "an embodiment," or similar language means that a particular feature, structure, or characteristic that is described in connection with the embodiment is included in at least one embodiment of the present disclosure. Thus, appearances of the phrases "in one embodiment," "in an embodiment," and similar language throughout this specification may, but do not necessarily, all refer to the same embodiment.

## Claims

1. A spinal endoscope (1) comprising an imaging device and an elongate rigid tube (100) coupled to the imaging device, wherein the tube (100) contains therein:
a lens (110) coupled to the imaging device to allow observation of a tissue;
first and second irrigation channels (120) to allow injection of a cleaning liquid to secure a view for surgery;
a working channel (130) to allow passage of a surgery-tool therethrough to a target location, and
an optical fiber (140) to allow irradiation of light beams,
the spinal endoscope (1) being **characterized in that** the working channel (130) includes a first portion (132) with a circular arc cross-sectional shape face-contacting and partially corresponding to an inner circumference of the elongate rigid tube (100), and a second portion (134) with a linear cross-sectional shape, wherein the second portion (134) connects both ends of the first portion (132), and
wherein the lens (110) is sandwiched between the first and the second irrigation channels (120), and
wherein the lens (110) contacts both irrigation channels (120), and
wherein the lens (110) and irrigation channels (120) all have a tangential contact with the inner circumference of the elongate rigid tube (100), and
wherein a remaining cross-sectional space excluding the spaces of the lens (110), the first and second irrigation channels (120), and the working channel (130) is filled with the optical fiber (140).

2. The spinal endoscope (1) of the preceding claim, wherein a length of the first portion (132) is at least about 70% of a total length of the inner circumference of the elongate rigid tube (100).

3. The spinal endoscope (1) of one of the preceding claims, wherein the cross-sectional area of the working channel (130) is larger than a sum of the cross-sectional areas of the lens (110), the irrigation channel (120), and the optical fiber 140.

4. The spinal endoscope (1) of one of the preceding claims, wherein the lens (110) has a circular cross-sectional shape.

5. The spinal endoscope (1) of one of the preceding claims, wherein the irrigation channel (120) has a circular cross-sectional shape.

6. The spinal endoscope (1) of one of the preceding claims, wherein the cross-sectional area of the working channel (130) is at least about 80% of the cross-sectional area of the elongate rigid tube (100).

7. The spinal endoscope (1) of one of the preceding claims, wherein a circular structure with the largest diameter among the circular structures such as the lens (110) and irrigation channels (120) has a tangential contact with the linear second portion (134) of the working channel (130).

8. The spinal endoscope (1) of the preceding claim, wherein the lens (110) has the tangential contact with the linear second portion (134) of the working channel (130).

9. The spinal endoscope (1) of one of the preceding claims, wherein the working channel (130) allows passage of a cage (CG) therethrough.

## Patentansprüche

1. Spinalendoskop (1) umfassend eine Abbildungsvorrichtung und ein langgestrecktes starres Rohr (100), das mit der Abbildungsvorrichtung gekoppelt ist, wobei das Rohr (100) darin enthält:
eine Linse (110), die mit der Abbildungsvorrichtung gekoppelt ist, um die Betrachtung eines Gewebes zu erlauben;
einen ersten und einen zweiten Spülkanal (120), um die Injektion einer Reinigungsflüssigkeit zu erlauben, um die Sicht bei einer Operation zu gewährleisten;
einen Arbeitskanal (130), um den Durchgang eines Operationswerkzeugs durch diesen hindurch zu einer Zielstelle zu erlauben, und
eine optische Faser (140), um eine Bestrahlung mit Lichtstrahlen zu erlauben,
wobei das Spinalendoskop (1) **dadurch gekennzeichnet ist, dass** der Arbeitskanal (130) einen ersten Abschnitt (132) mit einer kreisbogenförmigen Querschnittsform, die einen Innenumfang des langgestreckten starren Rohrs (100) flächig kontaktiert und ihr teilweise entspricht, und einen zweiten Abschnitt (134) mit einer linearen Querschnittsform umfasst, wobei der zweite Abschnitt (134) beide Enden des ersten Abschnitts (132) verbindet, und
wobei die Linse (110) sandwichartig zwischen dem ersten und dem zweiten Spülkanal (120) angeordnet ist, und
wobei die Linse (110) beide Spülkanäle (120) kontaktiert, und
wobei die Linse (110) und die Spülkanäle (120) alle einen tangentialen Kontakt mit dem Innenumfang des langgestreckten starren Rohrs (100) aufweisen, und
wobei ein verbleibender Querschnittsraum ohne die Räume der Linse (110), des ersten und des zweiten Spülkanals (120) und des Arbeitskanals (130) mit der optischen Faser (140) gefüllt ist.

2. Spinalendoskop (1) nach dem vorhergehenden Anspruch, wobei eine Länge des ersten Abschnitts (132) wenigstens etwa 70 % einer Gesamtlänge des Innenumfangs des langgestreckten starren Rohrs (100) beträgt.

3. Spinalendoskop (1) nach einem der vorhergehenden Ansprüche, wobei der Querschnittsbereich des Arbeitskanals (130) größer als eine Summe der Querschnittsbereiche der Linse (110), des Spülkanals (120) und der optischen Faser (140) ist.

4. Spinalendoskop (1) nach einem der vorhergehenden Ansprüche, wobei die Linse (110) eine kreisförmige Querschnittsform aufweist.

5. Spinalendoskop (1) nach einem der vorhergehenden Ansprüche, wobei der Spülkanal (120) eine kreisförmige Querschnittsform aufweist.

6. Spinalendoskop (1) nach einem der vorhergehenden Ansprüche, wobei der Querschnittsbereich des Arbeitskanals (130) wenigstens etwa 80 % des Querschnittsbereichs des langgestreckten starren Rohrs (100) beträgt.

7. Spinalendoskop (1) nach einem der vorhergehenden Ansprüche, wobei eine kreisförmige Struktur mit dem größten Durchmesser unter den kreisförmigen Strukturen, wie etwa der Linse (110) und den Spülkanälen (120), einen tangentialen Kontakt mit dem linearen zweiten Abschnitt (134) des Arbeitskanals (130) aufweist.

8. Spinalendoskop (1) nach dem vorhergehenden Anspruch, wobei die Linse (110) den tangentialen Kontakt mit dem linearen zweiten Abschnitt (134) des Arbeitskanals (130) aufweist.

9. Spinalendoskop (1) nach einem der vorhergehenden Ansprüche, wobei der Arbeitskanal (130) den Durchgang eines Käfigs (CG) durch ihn hindurch erlaubt.

## Revendications

1. Endoscope rachidien (1) comportant un dispositif d'imagerie et un tube rigide allongé (100) couplé au dispositif d'imagerie, dans lequel le tube (100) contient dans celui-ci :
- une lentille (110) couplée au dispositif d'imagerie pour permettre l'observation d'un tissu ;
- des premier et second canaux d'irrigation (120) pour permettre l'injection d'un liquide de nettoyage afin de garantir une vue pour la chirurgie ;
- un canal de travail (130) pour permettre le passage d'un outil chirurgical à travers celui-ci jusqu'à un emplacement cible, et
- une fibre optique (140) pour permettre l'irradiation de faisceaux lumineux,
l'endoscope rachidien (1) étant **caractérisé en ce que** le canal de travail (130) comprend une première partie (132) avec une forme d'arc circulaire en coupe transversale venant en contact avec la face et correspondant partiellement à une circonférence intérieure du tube rigide allongé (100), et une seconde partie (134) ayant une forme linéaire en coupe transversale, la seconde partie (134) reliant les deux extrémités de la première partie (132), et
où la lentille (110) est prise en sandwich entre le premier et le second canal d'irrigation (120), et
où la lentille (110) vient en contact avec les deux canaux d'irrigation (120), et
où la lentille (110) et les canaux d'irrigation (120) ont tous un contact tangentiel avec la circonférence intérieure du tube rigide allongé (100), et
où un espace résiduel en coupe transversal excluant les espaces de la lentille (110), les premier et second canaux d'irrigation (120), et le canal de travail (130) étant rempli avec la fibre optique (140).

2. Endoscope rachidien (1) selon la revendication précédente, dans lequel une longueur de la première partie (132) représente environ au moins 70% d'une longueur totale de la circonférence intérieure du tube rigide allongé (100).

3. Endoscope rachidien (1) selon une des revendications précédentes, dans lequel la zone en coupe transversale du canal de travail (130) est plus grande qu'une somme des zones en coupe transversale de la lentille (110), du canal d'irrigation (120), et de la fibre optique (140).

4. Endoscope rachidien (1) selon une des revendications précédentes, dans lequel la lentille (110) a une forme circulaire en coupe transversale.

5. Endoscope rachidien (1) selon une des revendications précédentes, dans lequel le canal d'irrigation (120) a une forme circulaire en coupe transversale.

6. Endoscope rachidien (1) selon une des revendications précédentes, la zone en coupe transversale du canal de travail (130) représente environ au moins 80% de la zone en coupe transversale du tube rigide allongé (100).

7. Endoscope rachidien (1) selon une des revendications précédentes, dans lequel une structure circulaire avec le diamètre le plus grand parmi les structures circulaires tels que la lentille (110) et les canaux d'irrigation (120) a un contact tangentiel avec la seconde partie linéaire (134) du canal de travail (130).

8. Endoscope rachidien (1) selon la revendication précédente, dans lequel la lentille (110) a un contact tangentiel avec la seconde partie linéaire (134) du canal de travail (130).

9. Endoscope rachidien (1) selon une des revendications précédentes, dans lequel le canal de travail (130) permet le passage d'une cage (CG) à travers celui-ci.
